(19) Europäisches Patentamt
European Patent Office
Office européen des brevets

(11) **EP 1 782 044 B1**

(12) **EUROPÄISCHE PATENTSCHRIFT**

(45) Veröffentlichungstag und Bekanntmachung des Hinweises auf die Patenterteilung:
**09.01.2013 Patentblatt 2013/02**

(21) Anmeldenummer: **05778179.1**

(22) Anmeldetag: **26.08.2005**

(51) Int Cl.:
*G01N 21/64* (2006.01)     *A61B 3/12* (2006.01)

(86) Internationale Anmeldenummer:
**PCT/DE2005/001517**

(87) Internationale Veröffentlichungsnummer:
**WO 2006/021206 (02.03.2006 Gazette 2006/09)**

(54) **Verfahren und Vorrichtung zur Trennung und genauen Bestimmung lokal wirksamer Fluorophore eines Objektes**

Method and device for the separation and precise determination of the locally active fluorophore of an object

Procédé et dispositif pour la séparation et la détermination précise d'un fluorophore d'un objet agissant localement

(84) Benannte Vertragsstaaten:
**DE FR GB IT**

(30) Priorität: **26.08.2004 DE 102004042198**

(43) Veröffentlichungstag der Anmeldung:
**09.05.2007 Patentblatt 2007/19**

(73) Patentinhaber: **Heidelberg Engineering GmbH**
**69121 Heidelberg (DE)**

(72) Erfinder:
• **SCHWEITZER, Dietrich**
**07749 Neustadt/Orla (DE)**
• **SCHWEITZER, Frank**
**07749 Jena (DE)**
• **HAMMER, Martin**
**07749 Jena (DE)**

(74) Vertreter: **Schmitt, Meinrad**
**Reble & Klose**
**Rechts- und Patentanwälte**
**Konrad-Zuse-Ring 32**
**68163 Mannheim (DE)**

(56) Entgegenhaltungen:
DD-A5- 290 266     DE-A1- 19 907 479
DE-A1- 19 920 157     US-A- 5 813 987
US-A1- 2004 051 847

• **MARTIN-FERNANDEZ M L ET AL: "A HIGH SENSITIVITY TIME-RESOLVED MICROFLUORIMETER FOR REAL-TIME BIOLOGY" REVIEW OF SCIENTIFIC INSTRUMENTS, AMERICAN INSTITUTE OF PHYSICS. NEW YORK, US, Bd. 67, Nr. 10, 1. Oktober 1996 (1996-10-01), Seiten 3716-3721, XP000635832 ISSN: 0034-6748**
• **APPEL ROLAND K ET AL: "Ultrabroadband collection and illumination optics for Raman and photoluminescence spectroscopy in the 200–700 nm wave band" REVIEW OF SCIENTIFIC INSTRUMENTS, AMERICAN INSTITUTE OF PHYSICS, US, Bd. 73, Nr. 10, Oktober 2002 (2002-10), Seiten 3440-3448, XP012039401 ISSN: 0034-6748**
• **SASE I ET AL: "REAL TIME IMAGING OF SINGLE FLUOROPHORES ON MOVING ACTIN WITH AN EPIFLUORESCENCE MICROSCOPE" BIOPHYSICAL JOURNAL, NEW YORK, US, US, Bd. 69, Nr. 2, August 1995 (1995-08), Seiten 323-328, XP008002278 ISSN: 0006-3495**

**Beschreibung**

Die Erfindung betrifft ein Verfahren und eine Vorrichtung zur Trennung und genauen Bestimmung lokal wirksamer Fluorophore, die in einem Objekt, beispielsweise durch ein Schichtsystem oder durch sonstige Strukturbeschaffenheit, infolge optischer Fluoreszenzanregung emittieren sowie unter Anwendung des an sich bekannten Laser-Scanning-Prinzips ausgewertet werden sollen. Mit Laser-Scanning-Anordnungen erfolgt bei Fluoreszenzmessungen eine serielle punktförmige Anregung des fluoreszierenden Objektes, wobei das punktförmig angeregte Feld konfokal in eine Feldblende abgebildet wird, die vor einem Einzeldetektor angeordnet ist. Aus dem einzeln nacheinander von jedem Bildpunkt detektierten Licht erfolgt nach Durchgang durch ein Sperrfilter der Aufbau eines zweidimensionalen Bildes des fluoreszierenden Objektes. Durch dieses konfokale Prinzip wird der Einfluss von Fluoreszenz- oder Streulicht von Schichten, die sich nicht in der Fokalebene des Anregungs- oder Abbildungssystems befinden, reduziert.

Aus der DE 199 07 479 A1 sind ein Verfahren und eine Vorrichtung bekannt, wobei die gleichzeitige Messung von Fluoreszenzspektren in schlitzförmig ausgedehnten Bereichen eines Augenhintergrundes erfolgt, welche mit einer geteilten Feldblende beleuchtet und mit weiterer geometrisch und spektral geteilter Feldblende auf einem Spektrograph abgebildet werden. Es ist eine Weißlichtquelle zur Fluoreszenzanregung mittels eines Beleuchtungsstrahlengangs vorgesehen, welcher eine Aperturblende aufweißt. Die Aperturblende im Beleuchtungsstrahlengang und eine Aperturblende in einem Messstrahlengang werden direkt vor der Augenlinse abgebildet und der Augenhintergrund wird gleichmäßig ausgeleuchtet. Die Feldblende wird mittels einer Linse konfokal auf einen Eintrittsspalt derart abgebildet, dass jene schlitzförmig und konfokal zum Eintrittsspalt ausgebindet ist. Es liegt die Aufgabe zugrunde, unterschiedliche Fluorophore mit möglichst geringem Handhabungs- und Justieraufwand jeweils unter exakt gleichen optischgeometischen Bedingungen messen zu können, obgleich jedes Fluorophor in einem charakteristischen Wellenlängenbereich angeregt werden muss und das Fluoreszenzspektrum in einem zur Anregung angepassten Spektralbereich zu detektieren ist. Eine konfokale Anordnung steilt die vorbekannte Vorrichtung nicht dar. Aus der DD 290 266 A5 sind ein Verfahren und eine Anordnung zur getrennten reflektrome-tischen Erfassung der Anteile des Fluoreszenzlichtes in verschiedenen Schichten eines Körpers, beispielsweise im menschlichen Auge, bekannt. Die Anordnung umfasst einen Mess-strahlengang, einen Beleuchtungsstrahlengang sowie auf eine Fundusschicht abgebildete Feldblenden, welche im Messstrahlengang wechselbar angeordnet sind. Weiterhin ist aus der DE 199 20 A1 ein Imaging Spectrometer mit scannender Schlitzbeleuchtung eines Objektes bekannt, wobei ein am Augenhintergrund abgetasteter Bildstreifen nach spektraler Zerlegung der Reflexionsspektren am jeweiligen Ort längs des Bildstreifens detektiert wird. Die Beleuchtung erfolgt mit weißem Licht, wobei die Stoffparameter in Medien beliebiger optischer Dichte bestimmt werden sollen, insbesondere die Sauerstoffsättigung von Blut sowohl in dicken Gefäßen, deren Dicke größer als die Eindringtiefe des Mess- lichtes ist, als auch in kapillarem Gewebe.

In Kamerasystemen (Schweitzer et al.: "Die altersabhängige Makulopathie - Vergleichende Untersuchungen zwischen Patienten, deren Kindern und Augengesunden", Ophthalmologe 2000, 97: 84-90; Hammer et al.: "Bestimmung der Konzentrationsverteilung des Makulapigmentes aus Reflexions- und Fluoreszenzaufnahmen", Ophthalmologe 2003, 100, 8: 611 - 617), beispielsweise in einer Funduskamera zur Messung der Fluoreszenz des Augenhintergrundes, wird dieser großflächig zur Fluoreszenz angeregt, wobei zur Vermeidung des Einflusses von Fremdlicht (beispielsweise störendes Streulicht) einer dem zu untersuchenden Objekt vorgelagerten Schicht, in diesem Falle der Augenlinse, in der Aperturblendenebene des Auges das Bild einer Aperturblende vorhanden ist, die sich im Anregungsstrahlengang befindet und durch deren peripher durchlässigen Teil das Anregungslicht tritt. Im Messstrahlengang ist eine weitere Aperturblende angeordnet, die zur Gewährleistung einer fehlerarmen Abbildung zentral durchlässig ist und deren peripherer Teil das Anregungslicht sperrt. Die Aufnahme des Fluoreszenzlichtes erfolgt nach Durchgang durch ein Sperrfilter mit einem Bilddetektor.

In der Praxis ist das auszuwertende Fluorophor des Objekts häufig durch den besagten Schicht- oder sonstigen Strukturcharakter, der weitere Fluorophore enthalten kann, überlagert, welcher die Auswertung der Fluoreszenz des zu untersuchenden Fluorophors, insbesondere dessen Fluoreszenzabklingzeit, beeinflusst.

Ein mögliches Anwendungsgebiet betrifft die präzise Bestimmung der zeitaufgelösten Fluoreszenz des Augenhintergrundes, wobei die auszuwertenden Fluoreszenzabklingzeiten, insbesondere durch ebenfalls mitangeregte Fluoreszenz der vorgelagerten Augenlinse beeinflusst werden.

Die Erfindung ist jedoch nicht auf ophthalmologische und nicht auf medizinische Anwendungen beschränkt. Mögliche Anwendungsgebiete ergeben sich auch beim Einsatz von Laser-Scanning-Mikroskopen.

Bei der Untersuchung der optischen Eigenschaften in Schichtsystemen, beispielsweise bei der Messung der zeitaufgelösten Autofluoreszenz vom Augenhintergrund, beeinflussen die Absorptions-, Streu- und Fluoreszenzeigenschaften vorgelagerter Schichten, insbesondere die Autofluoreszenz der Augenlinse, die Messungen der zeitaufgelösten Autofluoreszenz des Augenhintergrundes. Da der Augenhintergrund aus mehreren Schichten aufgebaut ist, tritt auch dort eine Überlagerung der Fluoreszenz der einzelnen Schichten auf.

In Schweitzer et al.: "Tau-Mapping of the autofluorescence of the human ocular fundus" (SPIE Vol. 4164, 2000, 79-89 und DE 199 20 158 A1, "Verfahren und Anordnung zur Bestimmung von Fluorophoren an Objekten, insbesondere am lebenden Augenhintergrund") wurde gezeigt, dass die zweidimensionale Messung der zeitaufgelösten Fluoreszenz, insbesondere der Autofluoreszenz, am Augenhintergrund unter Beachtung der maximal zulässigen Lichtbelastung gegenwärtig nur mittels einer Kombination zwischen Laser Scanning Technik zur Abtastung des Objektes und zeitkorreliertem Einzelphotonenzählen als Nachweistechnik möglich ist.

Die Messung der Fluoreszenzabklingzeit des Augenhintergrundes unter Anwendung einer großflächigen Beleuchtung des Augenhintergrundes mittels Funduskamera und unter Anwendung vom Messverfahren in der Frequenzdomäne erfordert Lichtbelastungen am Augenhintergrund, die dessen maximal zulässige Lichtbelastung überschreiten und damit zur Schädigung des Auges führen.

Im Gerät Fluorotron der Fa. Ocumetrix/USA kann die Fluoreszenz von Markern, z. B. von Natriumfluoreszin, in verschiedenen Schichten des Auges gemessen werden, indem das Volumen im Auge, das gemeinsam im Anregungsstrahlengang und im Messstrahlengang vorhanden ist, durch Verändern des Einfallswinkels des Anregungsstrahlengangs variabel gestaltet werden kann. Dieses Prinzip ist auf die Detektion der Fluoreszenz eines variablen Volumenelements längs der optischen Achse des Auges beschränkt.

Durch das in der Laser Scanning Technik angewandte konfokale Prinzip zwischen Abbildung eines Leuchtpunktes auf das Objekt und dessen Abbildung auf eine Konfokalblende vor einem Detektor wird der Einfluss des Reflexionslichtes von den vorderen Medien des Auges entsprechend Gleichung (1) um den Faktor Fr reduziert:

$$F_r = \frac{d^2_{Apertur}}{d^2_{Feld}} \qquad\qquad (1)$$

mit $d_{Apertur}$ -Durchmesser der im Auge wirksamen Aperturblende und $d_{Feld}$ -Durchmesser der Feldblende am Augenhintergrund Bei einem Durchmesser der Augenpupille von 6 mm und einem Feldblendendurchmesser von 0,1 mm am Augenhintergrund wird das Reflexionslicht der vorderen Augenmedien um den Faktor 3600 gegenüber dem Reflexionslicht vom Augenhintergrund reduziert.

Bei Messungen der Autofluoreszenz vom Augenhintergrund sind zusätzliche Betrachtungen notwendig. Das kurzwellige Anregungslicht regt nicht nur die Fluorophore am Fundus, sondern auch in den vorderen Augenmedien (Hornhaut, Linse) zur Fluoreszenz an. Die Fluoreszenzintensität ist im Allgemeinen proportional zur Absorption des Anregungslichtes. Diese ist wiederum proportional zur Extinktion.

Die Extinktion ist das Produkt aus Konzentration, Schichtdicke und substanzspezifischem Extinktionskoeffizienten. Liegt eine gleiche Konzentration des gleichen Fluorophors in den vorderen Augenmedien und im Fundus vor, so verhalten sich die Fluoreszenzintensitäten wie die Schichtdicken von Fundus und vorderen Augenmedien. Bei einer Dicke der fluoreszierenden Fundusschichten von 0,1 mm und einer Linsendicke von ca. 4 mm ist die Fluoreszenzintensität der Linse um den Faktor 40 höher als die Fundusfluoreszenz. Damit ist in diesem Beispiel die Unterdrückung der Linsenfluoreszenz durch das konfokale Prinzip auf den Faktor 90 reduziert. Durch die Absorption des Anregungslichtes in den vorderen Augenmedien ist die Intensität des Anregungslichtes am Fundus weiterhin reduziert. Mit zunehmendem Alter und bei Vorliegen einer Katarakt tritt weiterhin eine Streuung des Anregungslichtes in der Linse auf, die zu einer weiteren Verringerung des Anregungslichtes am Fundus führt. Für die Anregungswellenlänge 446 nm beträgt die Transmission der Okularmedien einer 20-jährigen Person ca. 50 %. Dieser Wert verringert sich mit zunehmendem Alter (Pokorny J. et al.: Aging of the human Lens, Appl. Opt. 1987, 26, 1437-1440). Unter Berücksichtigung dieser Einflüsse ist mit einem Anteil der Linsenfluoreszenz von einigen Prozent an der vom gesamten Auge gemessenen Fluoreszenz zu rechnen. Nach eigenen Messungen liegt die monoexponentielle Abklingzeit der natürlichen Augenlinse in der Größenordnung von 4,5 ns. Die bi-exponentielle Approximation der Autofluoreszenz des Augenhintergrundes liefert eine kurze Komponente τ1 von einigen hundert Pikosekunden, die mit einer relativen Amplitude von ca. 95 % gegenüber der langen Komponente τ2 von einigen Nanosekunden dominiert.

Es ist davon auszugehen, dass insbesondere die lange Komponente der Fundusfluoreszenz durch die Überlagerung mit der Linsenfluoreszenz beeinflusst ist.

Der Erfindung liegt die Aufgabe zu Grunde, lokal und zweidimensional in einem Objekt, beispielsweise im Auge, auftretende Fluorophore weitgehend unbeeinflusst von benachbarten störenden Fluoreszenzen mit hoher Genauigkeit und möglichst geringer Strahlenbelastung des Objektes unter Anwendung des an sich bekannten Laser-Scanner-Prinzips bestimmen zu können.

Erfindungsgemäß wird in einem Laser-Scanning-System der zur Fluoreszenzanregung dienende Beleuchtungsstrahlengang durch eine zusätzliche erste Aperturblende, welche in die Ebene eines zu unterdrückenden, d. h. nicht bei der Fluoreszenzauswertung des Objekts zu berücksichtigenden, Fluorophors abgebildete wird, ausschließlich in einem ersten Teilbereich, beispielsweise seinem zentralen (achsnahen) Bereich, wirksam und im übrigen Teilbereich, beispielsweise in seinem peripheren Bereich, unwirksam gehalten. Des Weiteren wird der zur Fluoreszenzauswertung dienende Messstrahlengang durch eine in die Ebene des zu unterdrückenden Fluorophors abgebildete zweite Aperturblende lediglich in einem Teilbereich, der in der Ebene des zu unterdrückenden Fluorophors für den Beleuchtungsstrahlengang unwirksam ist, beispielsweise dem peripheren Bereich, durchlässig gehalten, jedoch im Teilbereich, welcher für den Beleuchtungsstrahlengang wirksam ist, beispielsweise dem achsnahen Bereich, unterdrückt.

Auf diese Weise gelangt nur dasjenige Fluoreszenzlicht zur Auswertung, welches im gemeinsamen Teil von Beleuchtungs- und Messstrahlengang entsteht, wobei auch bei Anwendung des an sich bekannten Laser-Scanner-Prinzips ein für die Auswertung störendes Fluoreszenzlicht (benachbartes Fluorophor des zu untersuchenden Objekts, beispielsweise das Fluorophor der Augenlinse bei der Untersuchung des Augenhintergrundes) eliminiert ist.

Damit kann die Fluoreszenz, eines Objektes insbesondere die zeitaufgelöste Fluoreszenz des Augenhintergrundes, unbeeinflusst von der störenden Fluoreszenz benachbarter Fluorophore, insbesondere der Fluoreszenz der Augenlinse bei Fluoreszenzmessungen am Augenhintergrund, gemessen werden und daraus die für das zu bestimmende Fluorophor charakteristische Fluoreszenzlebensdauer bestimmt werden.

Werden die genannten zusätzlichen Aperturblenden im entsprechenden Beleuchtungs- bzw. Messstrahlengang jeweils verschiebbar angeordnet, kann die Abbildung der Aperturblende jeweils variabel auf die Ebene des bei der Fluoreszenzauswertung zu unterdrückenden Fluorophors eingestellt und verändert werden.

Die Teilbereiche des Beleuchtungs- und Messstrahlengangs können beispielsweise im Wesentlichen axial oder nicht axial durch die Aperturblenden getrennt werden. Eine nicht axiale Trennung könnte in gegenüberliegende Teilbereiche, z. B. in einen oberen und unteren Teilbereich oder in einen linken und rechten Teilbereich, erfolgen.

Im Falle einer axialen Trennung der Teilbereiche kann zur Messung des Augenhintergrundes die achsnahe zentrale Beleuchtungsapertur auf Grund der hohen Leuchtdichte des Lasers beispielsweise einen Durchmesser von 1 mm aufweisen. Die Aperturblende im Messstrahlengang kann dabei als Ringblende mit einem inneren Ring von beispielsweise 2 mm und einem äußeren Ring von beispielsweise 7 mm ausgeführt sein, wodurch bei isotroper Verteilung des Fluoreszenzlichtes vom Augenhintergrund weniger als 10 % des vom Objekt (Augenhintergrund) detektierbaren Fluoreszenzlichtes ausgeblendet werden, dafür aber das Fluoreszenzlicht der Augenlinse nahezu vollständig abgeschattet ist.

Die Erfindung soll nachstehend anhand eines in der Zeichnung dargestellten Ausführungsbeispiels näher erläutert werden.

Es zeigen:

Fig. 1: ophthalmologische Laser-Scanning-Anordnung zur Anregung und Auswertung der Fluoreszenz des Augenhintergrundes

Fig. 2: erfindungsgemäße Aperturblenden AP1 und AP2 im Beleuchtungs- bzw. Messstrahlengang der Anordnung gemäß Fig. 1

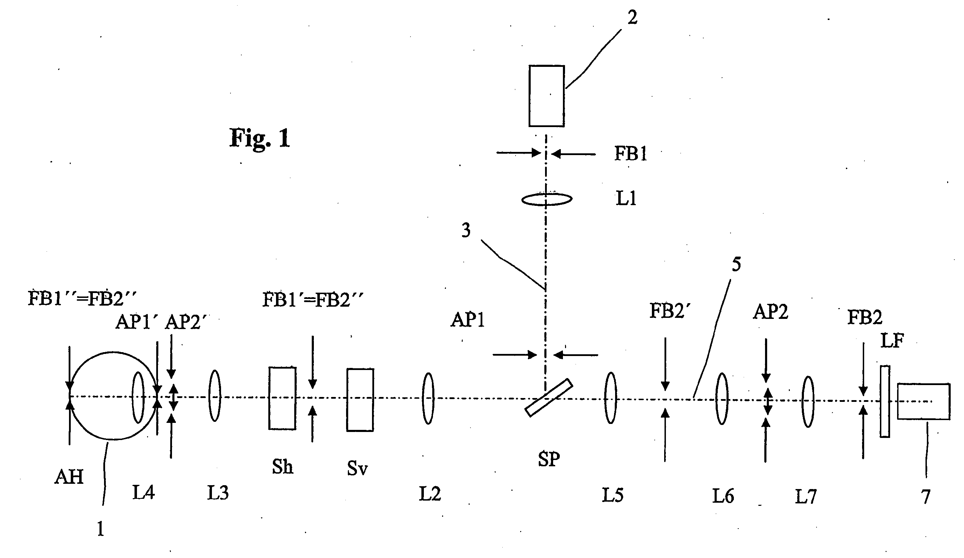

Fig. 1 zeigt eine ophthalmologische Laser-Scanning-Anordnung zur Anregung und Auswertung der Fluoreszenz des Augenhintergrundes AH eines Auges 1. Ein von einem Laser 2 ausgesendeter Beleuchtungsstrahlengang 3 wird über einen dichroitischen Spiegel SP auf das Auge 1 gelenkt. In diesem Beleuchtungsstrahlengang 3 befindet sich eine Feldblende FB1 in der Brennebene einer Linse L1, welche die Feldblende FB1 ins Unendliche abbildet. Ein Bild FB1' der Feldblende FB1 entsteht für den Beleuchtungsstrahlengang 3 in der bildseitigen Brennebene einer Linse L2 und in der objektseitigen Brennebene einer Linse L3, welche die Feldblende ins Unendliche abbildet.

Eine Augenlinse L4 des Auges 1 bildet das Bild FB1' der Feldblende FB1 als Bild FB1" aus dem Unendlichen auf den zu untersuchenden Augenhintergrund AH ab.

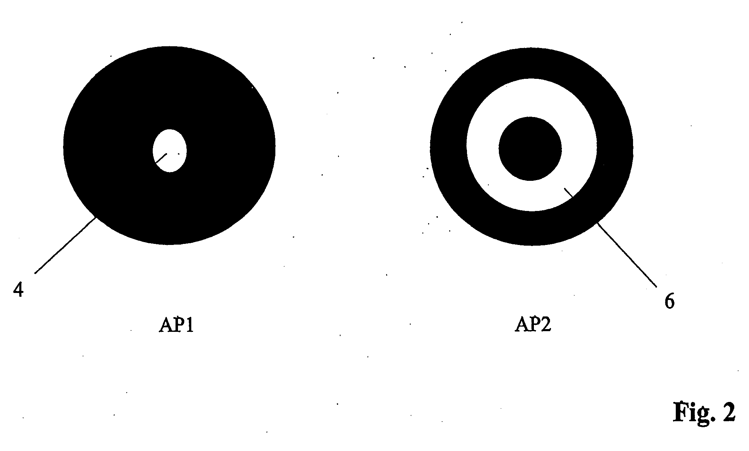

In der objektseitigen Brennebene der Linse L2 ist eine Aperturblende AP1 im Beleuchtungsstrahlengang 3 angeordnet, wodurch Linse L2 diese ins Unendliche abbildet. Die Aperturblende AP1, die lediglich in einem zentralen, achsnahen Bereich 4 durchlässig ist, ist im Querschnitt in Fig. 2 dargestellt. Linse L3 bildet die Aperturblende AP1 als Bild AP1' aus dem Unendlichen in deren bildseitige Brennebene ab, in der sich die Aperturebene des Auges 1 befindet.

In dieser Ebene befindet sich auch das Bild AP2' einer Aperturblende AP2, die in einem Messstrahlengang 5 des Laser-Scanning-Prinzips angeordnet ist. Wie in Fig. 2 dargestellt, ist die Aperturblende AP2 nur in einem Ringbereich 6 durchlässig. Dadurch blockt die Aperturblende AP2 das Reflexions- und Fluoreszenzlicht, das von den vorderen Augenmedien, insbesondere von der Augenlinse L4 stammt.

Das Fluoreszenzlicht und das diffus gestreute Licht des Augenhintergrundes AH treten durch das Bild der ringförmige Aperturblende AP 2', durchlaufen zwei Scanner Sh und Sv zur Horizontal- bzw. Vertikalablenkung und treffen auf den dichroitischen Spiegel SP. Dieser reflektiert das gestreute Licht, das die gleiche Wellenlänge wie das Anregungslicht hat. Für das langwelligere Fluoreszenzlicht, das in den Messstrahlengang 5 eintritt, hat der dichroitischen Spiegel SP eine hohe Transmission. Die Aperturblende AP2 ist in der Brennebene einer Linse L6 angeordnet. Das Bild der Aperturblende AP2 entsteht in der Brennebene einer Linse L5, in der sich auch die Brennebene der Linse L2 befindet. Die weitere Abbildung der Aperturblende AP2 in die Aperturebene des Auges erfolgt in der gleichen Weise wie für die Aperturblende AP1 beschrieben. Eine Feldblende FB2, die sich vor einem Detektor 7 im Messstrahlengang 5 in der Brennebene von Linse L7 befindet, wird über die Linsen L6, L5, L2 und L3 sowie die Augenlinse L4 konfokal zum Bild der Feldblende FB1 auf den Augenhintergrund AH des Auges 1 abgebildet.

Im Messstrahlengang 5 ist weiterhin ein Langpassfilter LF angeordnet, das zusätzlich das Anregungslicht aus dem Fluoreszenzlicht abblockt. An den Detektor 7 der Laser-Scanning-Anordnung sind eine aus Übersichtsgründen nicht in der Zeichnung dargestellte Einheit zur Messung der zeitaufgelösten Fluoreszenz sowie eine Einheit zur Bestimmung der Fluoreszenzabklingzeit angeschlossen.

In einer weiteren vorteilhaften Ausführung könnte an Stelle des dichroitischen Spiegels SP ein Spiegel, insbesondere ein Oberflächenspiegel, (nicht in der Zeichnung dargestellt) angeordnet sein, der das Licht des Beleuchtungsstrahlengangs 3 auf das Auge 1 lenkt und zugleich eine Aperturblendenfunktion sowohl für den Beleuchtungsstrahlengang 3 als auch für den Messstrahlengang 5 ausübt, indem der vom ebenfalls mitangeregten störenden Fluorophor beeinflusste Teil des Messstrahlengangs 5, beispielsweise dessen zentraler (achsnaher) Bereich, ausgeblendet wird. Der Beleuchtungsstrahlengang 3 wäre in seinem Außendurchmesser zuvor so zu begrenzen, dass kein gestreutes Anregungslicht in den Messstrahlengang 5 gelangen kann.

Die in den Ausführungsbeispielen dargestellten Linsen L1, L2, L3, L5, L6 und L7 können prinzipiell auch durch andere optische Abbildungselemente, beispielsweise durch entsprechende Spiegel, realisiert sein.

Bezugszeichenliste

| | |
|---|---|
| 1 | - Auge |
| 2 | - Laser |
| 3 | - Beleuchtungsstrahlengang |
| 4 | - Bereich (durchlässiger Bereich von AP1) |
| 5 | - Messstrahlengang |
| 6 | - Ringbereich (durchlässiger Bereich von AP2) |
| 7 | - Detektor |
| AH | - Augenhintergrund |
| L1, L2, L3, L5, L6, L7 | - Linse |
| L4 | - Augenlinse |
| SP | - dichroitischer Spiegel |
| FB1, FB2 | - Feldblende |
| FB1'; FB1" | - Bild der Feldblende FB1 |
| FB2', FB2" | - Bild der Feldblende FB2 |
| AP1, AP2 | - Aperturblende |
| AP1' | - Bild der Aperturblende AP1 |
| AP2' | - Bild der Aperturblende AP2 |
| Sh, Sv | - Scanner |
| LF | - Langpassfilter |

**Patentansprüche**

1. Verfahren zur Trennung und genauen Bestimmung lokal wirksamer Fluorophore eines Objektes, insbesondere des lebenden Auges, bei welchem das Objekt oder zumindest Teile davon durch einen von einer konfokal auf die Objektebene des auszuwertenden Fluorophors abgebildeten Feldblende begrenzten Beleuchtungsstrahlengang eines Lasers zur Fluoreszenz angeregt werden und bei welchem das infolge Fluoreszenzanregung entstehende und ggf. durch störende benachbarte Fluoreszenz überlagerte Fluoreszenzlicht in einem Messstrahlengang, welcher ebenfalls von einer konfokal auf die Objektebene abgebildeten Feldblende begrenzt ist, von einem Detektor registriert wird und das auszuwertende Fluorophor bestimmt wird, wobei der Beleuchtungsstrahlengang eines nicht bei der Auswertung zu berücksichtigenden Fluorophors in einem ersten Teilbereich wirksam und in einem weiteren davon örtlich getrennten Teilbereich unwirksam gehalten wird und wobei der Messstrahlengang eines nicht bei der Auswertung zu berücksichtigenden Fluorophors lediglich in einem Teilbereich, der für den Beleuchtungsstrahlengang unwirksam ist, durchlässig gehalten, jedoch im Teilbereich, welcher für den Beleuchtungsstrahlengang wirksam ist, unterdrückt wird,

   **dadurch gekennzeichnet, dass** die Detektion in einer konfokalen Laser Scanner Anordnung erfolgt, dass der Beleuchtungsstrahlengang in einer Ebene des nicht bei der Auswertung zu berücksichtigenden Fluorophors im ersten Teilbereich wirksam und in dem weiteren davon örtlich getrennten Teilbereich unwirksam gehalten wird und dass der Messstrahlengang in der Ebene des nicht bei der Auswertung zu berücksichtigenden Fluorophors lediglich im Teilbereich, der für den Beleuchtungsstrahlengang unwirksam ist, durchlässig gehalten, jedoch im Teilbereich, welcher für den Beleuchtungsstrahlengang wirksam ist, unterdrückt wird.

2. Verfahren gemäß Anspruch 1, **dadurch gekennzeichnet, dass** das auszuwertende Fluorophor unter Auswertung der jeweiligen Fluoreszenzabklingzeit bestimmt wird.

3. Verfahren gemäß Anspruch 1 oder 2, **dadurch gekennzeichnet, dass** der erste Teilbereich ein achsnaher Bereich und der zweite Teilbereich ein peripherer Bereich des Beleuchtungsstrahlengangs ist.

4. Verfahren gemäß einem der Ansprüche 1 bis 3, **dadurch gekennzeichnet, dass** die korrespondierenden wirksamen und unwirksamen bzw. durchlässigen und undurchlässigen Teilbereiche des Beleuchtungs- und Messstrahlengangs jeweils im Wesentlichen axial in einen achsnahen zentralen Bereich und in einen peripheren Bereich getrennt werden.

5. Verfahren gemäß Anspruch 1 oder 2, **dadurch gekennzeichnet, dass** die korrespondierenden wirksamen und unwirksamen bzw. durchlässigen und undurchlässigen Teilbereiche des Beleuchtungs- und Messstrahlengangs jeweils nicht axial, beispielsweise in einen oberen und unteren Bereich bzw. in einen linken und rechten Bereich, getrennt werden.

6. Verfahren gemäß Anspruch 1 oder 2, **dadurch gekennzeichnet, dass** die korrespondierenden wirksamen und unwirksamen bzw. durchlässigen und undurchlässigen Teilbereiche des Beleuchtungs- und Messstrahlengangs jeweils in gleich große Bereiche getrennt werden.

7. Verfahren gemäß Anspruch 1 oder 2, **dadurch gekennzeichnet, dass** die korrespondierenden wirksamen und unwirksamen bzw. durchlässigen und undurchlässigen Teilbereiche des Beleuchtungs- und Messstrahlengangs zugunsten einer Unterdrückung des störenden Fluorophors im Messstrahlengang jeweils in nicht gleich große Bereiche getrennt werden.

8. Vorrichtung zur Trennung und genauen Bestimmung lokal wirksamer Fluorophore eines Objektes (1), insbesondere des lebenden Auges, umfassend einen Laser (2), eine Konfokale Laser Scanner Anordnung, eine Feldblende (FB1) und eine Aperturblende (AP1) in einem Beleuchtungsstrahlengang (3) zwischen dem Laser (2) und dem Objekt (1), einen Detektor (7) sowie ferner Aperturblende (AP2) in einem Messstrahlengang (5) zwischen dem Objekt (1) und dem Detektor (7), wobei das Objekt (1) oder zumindest Teile davon durch den von der konfokal auf die Objektebene des auszuwertenden Fluorophors abgebildeten Feldblende (FB1) begrenzten Beleuchtungsstrahlengang (3) des Lasers (2), zur Fluoreszenz angeregt werden und wobei das infolge der Fluoreszenzanregung entstehende und ggf. durch störende benachbarte Fluoreszenz überlagerte Fluoreszenzlicht in dem Messstrahlengang (5), welcher zwischen dem Objekt (1) und dem Detektor (7) vorhanden ist und ebenfalls von einer konfokal auf die Objektebene abgebildeten Feldblende (FB2) begrenzt ist, von dem Detektor (7) registriert wird und das auszuwertende Fluorphor bestimmt wird, wobei der Detektor (7) in der Konfokalen Laser-Scanner-Anordnung angeordnet ist, wobei im Beleuchtungsstrahlengang (3) die in der Ebene eines nicht bei der Auswertung zu berücksichtigenden Fluorophors abgebildete Aperturblende (AP1) angeordnet ist welche ausschliezlich in einem ersten Teilbereich (4) durchlässig und im übrigen Teilbereich für den Beleuchtungsstrahlengang (3) undurchlässig ist und wobei im Messstrahlengang (5) die ebenfalls in der Ebene des nicht bei der Auswertung zu berücksichtigenden Fluorophors abgebildete Aperturblende (AP2) vorgesehen ist, welche lediglich in dem Teilbereich (6), der für den Beleuchtungsstrahlengang (3) in der Ebene des nicht bei der Auswertung zu berücksichtigenden Fluorophors unwirksam ist, durchlässig und im übrigen Teilbereich für den Messstrahlengang (5) undurchlässig ist.

9. Vorrichtung gemäß Anspruch 8, **dadurch gekennzeichnet, dass** mittels des Detektors (7) unter Auswertung der jeweiligen Fluoreszendabklingzeit das auszuwertende Fluorophor bestimmbar ist.

10. Vorrichtung gemäß Anspruch 8 oder 9, **dadurch gekennzeichnet, dass** die Aperturblenden (AP1 und AP2) im Beleuchtungsstrahlengang (3) bzw. im Messstrahlengang (5) jeweils im Wesentlichen axial getrennte Teilbereiche und somit jeweils einen achsnahen (4) und einen achsperipheren (6) Teilbereich zur Strahltrennung aufweisen.

11. Vorrichtung gemäß Anspruch 8 oder 9, **dadurch gekennzeichnet, dass** die Aperturblenden (AP1 und AP2) im Beleuchtungsstrahlengang (3) bzw. im Messstrahlengang (5) nicht axial, sondern beispielsweise jeweils in einen oberen und unteren Bereich bzw. in einen linken und rechten Bereich getrennte Teilbereiche zur Strahltrennung aufweisen.

12. Vorrichtung gemäß Anspruch 8 oder 9, **dadurch gekennzeichnet, dass** die Aperturblenden (AP1 und AP2) im Beleuchtungsstrahlengang (3) bzw. im Messstrahlengang (5) jeweils gleich große Teilbereiche zur Strahltrennung aufweisen.

13. Vorrichtung gemäß Anspruch 8 oder 9, **dadurch gekennzeichnet, dass** die Aperturblenden (AP1 und AP2) im Beleuchtungsstrahlengang (3) bzw. im Messstrahlengang (5) zugunsten einer Unterdrückung des störenden Fluorophors durch die Aperturblende (AP2) im Messstrahlengang (5) jeweils nicht gleich große Teilbereiche zur Strahltrennung aufweisen.

**Claims**

1. A method for separating and precisely determining locally active fluorophores of an object, in particular the living eye, in which the object or at least parts thereof is excited to fluorescence by means of a light beam from a laser limited by a field diaphragm confocally imaged onto the object plane of the fluorophore to be evaluated and wherein the fluorescence light resulting from fluorescence excitation, possibly superimposed with perturbing collateral fluorescence, is registered by a detector in a measuring beam, which is also limited by a field diaphragm confocally imaged onto the object plane and the fluorophore to be evaluated is determined, wherein the light beam of a fluorophore that is not to be taken into account in the evaluation is active in a first zone and inactive in a further spatially separate zone, and wherein the measuring beam of a fluorophore that is not to be taken into account in the evaluation is only transparent to the light beam in a zone that is inactive, but is suppressed in the zone which is active for the light beam,
   **characterized in that** detection is carried out in a confocal laser scanner system, **in that** the light beam is active in a plane of the fluorophore that is not to be taken into account in the evaluation in the first zone and is inactive in the further spatially separate zone and **in that** in the plane of the fluorophore that is not to be taken into account in the evaluation, the measuring beam is only transparent in the zone that is inactive for the light beam, but is suppressed in the zone that is active for the light beam.

2. The method as claimed in claim 1, **characterized in that** the fluorophore to be evaluated is determined by evaluating the appropriate fluorescence decay time.

3. The method as claimed in claim 1 or claim 2, **characterized in that** the first zone is a paraxial zone and the second zone is a peripheral zone of the light beam.

4. The method as claimed in one of claims 1 to 3, **characterized in that** the corresponding active and inactive or transparent and obscure zones of the light beam and measuring beam are respectively essentially axially separated into a paraxial central zone and a peripheral zone.

5. The method as claimed in claim 1 or claim 2, **characterized in that** the corresponding active and inactive or transparent and obscure zones of the light beam and measuring beam are each separated non-axially, for example into an upper and lower zone or into a left and right zone.

6. The method as claimed in claim 1 or claim 2, **characterized in that** the corresponding active and inactive or transparent and obscure zones of the light beam and measuring beam are each separated into zones of equal size.

7. The method as claimed in claim 1 or claim 2, **characterized in that** the corresponding active and inactive or transparent and obscure zones of the light beam and measuring beam are respectively separated into zones of unequal sizes for the purposes of suppressing the perturbing fluorophore in the measuring beam.

8. A device for separating and precisely determining locally active fluorophores of an object (1), in particular the living eye, comprising a laser (2), a confocal laser scanner system, a field diaphragm (FB1) and an aperture diaphragm (AP1) in a light beam (3) between the laser (2) and the object (1), a detector (7) as well as a further aperture diaphragm (AP2) in a measuring beam (5) between the object (1) and the detector (7), wherein the object (1) or at least parts thereof is excited to fluorescence by the light beam (3) of the laser (2) limited by the field diaphragm (FB1) confocally imaged onto the object plane of the fluorophore to be evaluated and wherein the fluorescence light resulting from fluorescence excitation, possibly superimposed with perturbing collateral fluorescence in the measuring beam (5), which is between the object (1) and the detector (7) and is also limited by a field diaphragm (FB2) confocally imaged onto the object plane, is registered by the detector (7) and the fluorophore to be evaluated is determined,

wherein the detector (7) is disposed in the confocal laser scanning system, wherein the aperture diaphragm (AP1) that is imaged in the plane of a fluorophore that is not to be taken into account in the evaluation is disposed in the light beam (3), which aperture diaphragm is only transparent to the light beam (3) in a first zone (4) and is obscure to the light beam (3) in the remaining zone and

wherein the aperture diaphragm (AP2) that is also imaged in the plane of the fluorophore that is not to be taken into account in the evaluation is disposed in the measuring beam (5), which aperture diaphragm is only transparent to the measuring beam (5) in the zone (6) which is inactive for the light beam (3) in the plane of the fluorophore that is not to be taken into account for the evaluation and is obscure to the measuring beam (5) in the remaining zone.

9. The device as claimed in claim 8, **characterized in that** the fluorophore to be evaluated can be determined by means of the detector (7) by evaluating the appropriate fluorescence decay time.

10. The device as claimed in claim 8 or claim 9, **characterized in that** the aperture diaphragms (AP1 and AP2) in the light beam (3) or in the measuring beam (5) each essentially comprise axially separated zones and thereby each have a paraxial zone (4) and a zone (6) which is distant from the axis for the purposes of beam separation.

11. The device as claimed in claim 8 or claim 9, **characterized in that** the aperture diaphragms (AP1 and AP2) in the light beam (3) or in the measuring beam (5) are not separated axially but are respectively, for example, separated into an upper and a lower zone or into a left and a right zone for the purposes of beam separation.

12. The device as claimed in claim 8 or claim 9, **characterized in that** the aperture diaphragms (AP1 and AP2) in the light beam (3) or in the measuring beam (5) each have equal sized zones for the purposes of beam separation.

13. The device as claimed in claim 8 or claim 9, **characterized in that** the aperture diaphragms (AP1 and AP2) in the light beam (3) or in the measuring beam (5) each have unequally sized zones for the purposes of beam separation in order to suppress the perturbing fluorophore by means of the aperture diaphragm (AP2) in the measuring beam (5).

## Revendications

1. Procédé pour la séparation et la détermination exacte de fluorophores agissant localement d'un objet, en particulier de l'oeil vivant, dans lequel l'objet ou du moins des parties de celui-ci est/sont excité(s) pour produire la fluorescence, par un trajet de rayons d'éclairage d'un laser, délimité par un diaphragme de champ projeté de façon confocale sur le plan d'objet du fluorophore à évaluer, et dans lequel la lumière fluorescente, produite par l'excitation par fluorescence et subissant éventuellement l'interférence d'une fluorescence voisine perturbante, est enregistrée par un détecteur dans un trajet de rayons de mesure également délimité par un diaphragme de champ projeté de façon confocale sur le plan d'objet, et le fluorophore à évaluer est déterminé, dans lequel le trajet de rayons d'éclairage d'un fluorophore exclu de l'évaluation est maintenu actif dans une première région partielle et inactif dans une autre région partielle spatialement séparée de la première, et dans lequel le trajet de rayons de mesure d'un fluorophore exclu de l'évaluation est maintenu perméable seulement dans une région partielle inactive pour le trajet de rayons d'éclairage, mais imperméable dans la région partielle active pour le trajet de rayons d'éclairage, **caractérisé en ce que** la détection est réalisée dans un ensemble laser-scanner confocal, **en ce que** dans un plan du fluorophore exclu de l'évaluation, le trajet de rayons d'éclairage est actif dans la première région partielle et maintenu inactif dans l'autre région partielle spatialement séparée de la première, et **en ce que** dans le plan du fluorophore exclu de l'évaluation, le trajet de rayons de mesure est maintenu perméable seulement dans la région partielle qui est inactive pour le trajet de rayons d'éclairage, mais imperméable dans la région partielle active pour le trajet de rayons d'éclairage.

2. Procédé selon la revendication 1, **caractérisé en ce que** le fluorophore à évaluer est déterminé par évaluation du temps d'affaiblissement correspondant.

3. Procédé selon la revendication 1 ou 2, **caractérisé en ce que** la première région partielle est une région proche de l'axe, et la deuxième région partielle est une région périphérique du trajet de rayons d'éclairage.

4. Procédé selon l'une des revendications 1 à 3, **caractérisé en ce que** les régions partielles correspondantes, actives et inactives ou perméables et imperméables, du trajet de rayons de mesure et d'éclairage sont séparées, essentiellement axialement, en une région centrale proche de l'axe et une région périphérique.

5. Procédé selon la revendication 1 ou 2, **caractérisé en ce que** les régions partielles correspondantes, actives et inactives ou perméables et imperméables, du trajet de rayons de mesure et d'éclairage sont séparées non pas axialement, par exemple en une région inférieure et une région supérieure, ou en une région de gauche et une région de droite.

6. Procédé selon la revendication 1 ou 2, **caractérisé en ce que** les régions partielles correspondantes, actives et inactives ou perméables et imperméables, du trajet de rayons de mesure et d'éclairage sont séparées respectivement en régions de même taille.

7. Procédé selon la revendication 1 ou 2, **caractérisé en ce que** les régions partielles correspondantes, actives et inactives ou perméables et imperméables, du trajet de rayons de mesure et d'éclairage sont séparées respectivement en régions de différentes tailles, au profit d'une suppression du fluorophore nuisible dans le trajet de rayons de mesure.

8. Dispositif pour la séparation et la détermination exacte de fluorophores agissant localement d'un objet (1), en particulier de l'oeil vivant, comprenant un laser (2), un ensemble confocal laser-scanner, un diaphragme de champ (FB1) et un diaphragme d'ouverture (AP1) dans un trajet de rayons d'éclairage (3) entre le laser (2) et l'objet (1), un détecteur (7) et un diaphragme d'ouverture (AP2) dans un trajet de rayons de mesure (5) entre l'objet (1) et le détecteur (7), dans lequel l'objet (1) ou du moins des parties de celui-ci est/sont excité(s) pour produire la fluorescence, par le trajet de rayons d'éclairage (3) du laser (2) délimité par le diaphragme de champ (FB1) projeté de façon confocale sur le plan d'objet du fluorophore à évaluer, et dans lequel la lumière fluorescente produite par l'excitation par fluorescence et subissant éventuellement l'interférence d'une fluorescence voisine perturbante est enregistrée par le détecteur (7), dans le trajet de rayons de mesure (5) présent entre l'objet (1) et le détecteur (7) et également délimité par un diaphragme de champ (FB2) projeté de façon confocale sur le plan d'objet, et le fluorophore à évaluer est déterminé,
dans lequel le détecteur (7) est agencé dans l'ensemble confocal laser-scanner, dans lequel le diaphragme d'ouverture (AP1) projeté dans le plan d'un fluorophore exclu de l'évaluation est agencé dans le trajet de rayons d'éclairage (3), tout en étant perméable exclusivement dans une première région partielle (4) et imperméable dans la région partielle restante pour le trajet de rayons d'éclairage (3),
et dans lequel le diaphragme d'ouverture (AP2) également projeté dans le plan d'un fluorophore exclu de l'évaluation est prévu dans le trajet de rayons de mesure (5), tout en étant perméable seulement dans la région partielle (6) qui est inactive pour le trajet de rayons d'éclairage (3) dans le plan d'un fluorophore exclu de l'évaluation, et imperméable dans la région partielle restante pour le trajet de rayons de mesure (5).

9. Dispositif selon la revendication 8, **caractérisé en ce que** le détecteur (7) permet de déterminer le fluorophore à évaluer, en évaluant respectivement le temps d'affaiblissement de la fluorescence.

10. Dispositif selon la revendication 8 ou 9, **caractérisé en ce que** dans le trajet de rayons d'éclairage (3) ou le trajet de rayons de mesure (5), les diaphragmes d'ouverture (AP1 et AP2) comportent respectivement des régions partielles séparées essentiellement axialement, et ainsi respectivement une région partielle proche de l'axe (4) et une région partielle périphérique (6), pour la séparation des rayons.

11. Dispositif selon la revendication 8 ou 9, **caractérisé en ce que** dans le trajet de rayons d'éclairage (3) ou le trajet de rayons de mesure (5), les diaphragmes d'ouverture (AP1 et AP2) comportent respectivement des régions partielles séparées non pas axialement, mais par exemple en une région supérieure et une région inférieure, ou en une région de gauche et une région de droite, pour la séparation des rayons.

12. Dispositif selon la revendication 8 ou 9, **caractérisé en ce que** dans le trajet de rayons d'éclairage (3) ou le trajet de rayons de mesure (5), les diaphragmes d'ouverture (AP1 et AP2) comportent respectivement des régions partielles de même taille, pour la séparation des rayons.

13. Dispositif selon la revendication 8 ou 9, **caractérisé en ce que** dans le trajet de rayons d'éclairage (3) ou le trajet de rayons de mesure (5), les diaphragmes d'ouverture (AP1 et AP2) comportent respectivement des régions partielles de tailles différentes, au profit d'une suppression du fluorophore nuisible par le diaphragme d'ouverture (AP2) dans le trajet de rayons de mesure (5), pour la séparation des rayons.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19907479 A1
- DD 290266 A5
- DE 19920 A1
- DE 19920158 A1

**In der Beschreibung aufgeführte Nicht-Patentliteratur**

- **SCHWEITZER et al.** Die altersabhängige Makulopathie - Vergleichende Untersuchungen zwischen Patienten, deren Kindern und Augengesunden. *Ophthalmologe,* 2000, vol. 97, 84-90
- **HAMMER et al.** Bestimmung der Konzentrationsverteilung des Makulapigmentes aus Reflexions- und Fluoreszenzaufnahmen. *Ophthalmologe,* 2003, vol. 100 (8), 611-617
- **SCHWEITZER et al.** Tau-Mapping of the autofluorescence of the human ocular fundus. *SPIE,* 2000, vol. 4164, 79-89
- **POKORNY J. et al.** Aging of the human Lens. *Appl. Opt.,* 1987, vol. 26, 1437-1440